# EUROPÄISCHE PATENTSCHRIFT

(11) **EP 4 204 883 B1**
(45) Veröffentlichungstag und Bekanntmachung des Hinweises auf die Patenterteilung: **22.07.2026**
(21) Anmeldenummer: 21827189.8
(22) Anmeldetag: 29.11.2021
(51) Int. Cl.: G02B 7/00, A61B 1/00, A61B 50/13, A61B 90/50, G02B 21/22, A61B 90/25, G02B 21/00

(54) **3D AUSGABEVORRICHTUNG ZUR STEREOSKOPISCHEN BILDWIEDERGABE**
3D OUTPUT APPARATUS FOR STEREOSCOPIC IMAGE REPRODUCTION
APPAREIL DE SORTIE 3D POUR LA REPRODUCTION D'IMAGES STÉRÉOSCOPIQUES

(30) Priorität: 30.11.2020 DE 102020131595
(43) Veröffentlichungstag der Anmeldung: 05.07.2023
(73) Patentinhaber: Blazejewski Medi-Tech GmbH, 79350 Sexau (DE)
(72) Erfinder: BLAZEJEWSKI, Reinhold, 79261 Gutach i.Br. (DE)
(74) Vertreter: Geitz Patentanwälte PartG mbB
(86) Internationale Anmeldenummer: PCT/DE2021/200222
(87) Internationale Veröffentlichungsnummer: WO 2022/111775

(56) Entgegenhaltungen:
- EP-A1- 2 044 902
- WO-A1-2016/069655
- DE-A1- 10 027 196
- DE-U1- 202016 101 003
- US-A1- 2007 156 122
- US-A1- 2011 080 536
- US-A1- 2018 092 706
- US-A1- 2019 377 149
- US-A1- 2020 297 195

## Beschreibung

Die Erfindung geht aus von einer 3D Ausgabevorrichtung zur stereoskopischen Bildwiedergabe.

3D Bildgebungssysteme sind beispielsweise in 3D Endoskopen oder 3D Mikroskopen enthalten. Sie dienen unter anderem der Untersuchung und Bearbeitung von Objekten und Strukturen in der Medizin, der Biologie, der Technik und der Materialwissenschaften. Als Kamera oder Bildsensor dient häufig ein Bildwandlerchip, beispielsweise CMOS oder CCD. Der Bildsensor, auch Bildgeber genannt, wandelt die optischen Signale in elektrische Signale um, welche anschließend auf einem Bildschirm oder einem Monitor für eine das Objekt untersuchende Person optisch sichtbar gemacht werden. Um ein dreidimensionales Bild zu erzeugen, werden mit dem 3D Bildgebungssystem Bilddaten für das linke Auge und das rechte Auge der Person getrennt aufgenommen. Hierzu dienen in der Regel einer linker Bildkanal und ein rechter Bildkanal.

Mit einer 3D Ausgabevorrichtung werden die mit dem 3D Bildgebungssystem aufgenommen Bilddaten so sichtbar gemacht, dass die Person einen dreidimensionalen Eindruck von dem untersuchten Objekt erhält. Hierzu müssen die Bilddaten für das linke Auge und die Bilddaten für das rechte Auge angezeigt werden. Die für das linke Auge vorgesehenen Bilddaten dürfen nur von dem linken Auge der Person wahrgenommen werden. Die für das rechte Auge vorgesehenen Bilddaten dürfen nur von dem rechten Auge der Person wahrgenommen werden. Hierzu sind verschiedene Geräte bekannt, beispielsweise 3D-Monitore, die sowohl die Bilddaten für das linke Auge als auch die Bilddaten für das rechte Auge anzeigen. Die das Objekt untersuchende Person benötigt in der Regel eine spezielle Brille, so dass die für das linke Auge bestimmten Bilddaten nur vom linken Auge wahrgenommen werden, und die für das rechte Auge bestimmten Bilddaten nur von dem rechten Auge der Person wahrgenommen werden. Zu derartigen Brillen zählen beispielsweise Polfilterbrillen, Farbfilterbrillen, Interferenzfilterbrillen und LCD-Shutterbrillen. Eine derartige Kombination aus einem Monitor und einer speziell von dem Betrachter zu tragenden Brille ist aus der US 2020/297195 A1 bekannt. Darüber hinaus sind spezielle Sichtgeräte bekannt, die am Kopf einer Person in unmittelbarer Nähe zu ihren Augen angeordnet sind. Derartige Sichtgeräte sind beispielsweise in ein 3D-Headset integriert. Sie werden auch als 3D-Video-Brillen bezeichnet und sind mit zwei Displays ausgestattet.

Die US 2018/092706 A1 offenbart ein immersives dreidimensionales Display, welches Bestandteil eines robotergestützten chirurgischen Systems ist. Das immersive Display umfasst einen mit mehreren Gelenken ausgestatteten Tragarm, ein Gehäuse, das an dem Tragarm montiert ist und an das ein Benutzer sein Gesicht anlegen kann, und mindestens zwei Okularbaugruppen, die in dem Gehäuse angeordnet sind, um eine dreidimensionale Anzeige zu ermöglichen. Der Tragarm dient dazu, das Gewicht des Gehäuses zu tragen und das Gehäuse relativ zu dem Benutzer zu positionieren. Der Tragarm kann an einer Sitzlehne oder Kopfstütze eines Sitzes für den Benutzer, an einer Decke, einer Wand, einer Säule oder einem Wagen befestigt sein. Nachteilig an dieser Vorrichtung ist jedoch, dass die spezifische mechanische Ausgestaltung des Gelenks, welches das Gehäuse mit dem Gelenkarm verbindet, nicht detailliert beschrieben ist. Es wird keine konkrete technische Lehre offenbart, wie eine vollständige, intuitive und mechanisch stabile dreidimensionale Feinausrichtung der Visualisierungseinrichtung auf kompakte Weise realisiert werden kann.

Die US 2011/080536 A1 und die EP 2 044 902 A1 offenbaren eine stereoskopische Bildanzeigevorrichtung für ein Operationsmikroskop. Die Bildanzeigevorrichtung umfasst ein in einem Gehäuse angeordnetes Paar von linken und rechten Elektronikbild-Anzeigepaneelen zum Anzeigen eines Paares von rechten und linken elektronischen Bildern. Die Bildanzeigevorrichtung ist an einem Tragarm beweglich aufgenommen, der über ein Verbindungsstück an einer Decke oder einer Stützstruktur aufgehängt ist. Die Dokumente zeigen zwar eine gelenkige Anbindung der jeweiligen Anzeigeeinheit, enthalten jedoch keine konkrete mechanische Realisierung des Kopfgelenks, über welches die Visualisierungseinrichtung mit dem Tragarm verbunden ist. Sie liefern somit keine Anregung, wie die finale, ergonomische Ausrichtung der Anzeigeeinheit am Kopf des Benutzers präzise und intuitiv gestaltet werden kann.

Die DE 100 27 196 A1 offenbart ein Chirurgie-Operationstischsystem, welches mit einem Stereomikroskop und mit einem Stereoskopbetrachtungsgerät ausgestattet ist. Dabei ist das Stereomikroskop an einem ersten Ständer angeordnet, während das Stereoskopbetrachtungsgerät an einem zweiten Ständer räumlich getrennt von dem ersten Ständer angeordnet ist. Das Stereoskopbetrachtungsgerät umfasst als Monitor einen LCD-Schirm. Eine linke Hälfte des LCD-Schirms zeigt ein linkes Bild des Stereomikroskops und eine rechte Hälfte des LCD-Schirms zeigt ein rechtes Bild an. Auch dieses Dokument offenbart keine konkrete mechanische Realisierung des Kopfgelenks, über welches die Visualisierungseinrichtung mit dem Tragarm verbunden ist.

Nachteilig bei den aus dem Stand der Technik bekannten 3D-Monitoren ist, dass diese aufgrund ihrer Größe räumlich getrennt von dem Einsatzort des Bildgebungssystem angeordnet werden müssen. Es ist daher für eine Person, unmöglich, sowohl den Einsatzort des Bildgebungssystems als auch den 3D-Monitor zu betrachten, ohne dabei den Kopf zu drehen und die Blickrichtung zu ändern. Darüber hinaus ist die Person durch das Tragen der notwendigen zusätzlichen Brille, wie Polfilterbrille oder ähnliche, eingeschränkt, da die Lichtintensität reduziert ist. Dies ist insbesondere beim Betrachten des Einsatzortes, beim manuellen Betätigen des Bildgebungssystems und beim Steuern eines eventuell zusätzlichen Instrumentes störend.

Nachteilig bei 3D-Headsets oder 3D-Videobrillen ist, dass diese am Kopf der Person befestigt werden und die Augen abdecken. Die Person kann den Einsatzort des Bildgebungssystems, das Bildgebungssystem selbst und ein eventuell zusätzliches Instrument nicht sehen, solange das Sichtgerät am Kopf der Person angeordnet ist. Möchte die Person das Sichtgerät selbst ablegen, so muss sie die Untersuchung des Objektes unterbrechen oder eine dritte Person bitten, das Sichtgerät vom Kopf der Person abzunehmen.

Ausgehend vom Stand der Technik besteht die Aufgabe der Erfindung darin, eine 3D Ausgabevorrichtung zur stereoskopischen Bildwiedergabe zur Verfügung zu stellen, mit der eine Person sowohl den Einsatzort des Bildgebungssystems als auch das von dem Bildgebungssystem aufgenommene 3D Bild des untersuchten Objektes sehen kann, ohne die Blickrichtung erheblich zu ändern, wobei die Anbindung der Visualisierungseinrichtung an den Gelenkarm so verbessert ist, dass eine vollständige und intuitive dreidimensionale Feinausrichtung der Visualisierungseinrichtung relativ zum Nutzerkopf ermöglicht wird.

Diese Aufgabe wird durch eine 3D Ausgabevorrichtung zur stereoskopischen Bildwiedergabe mit den Merkmalen des Anspruchs 1 gelöst. Die Ausgabevorrichtung zeichnet sich durch einen Gelenkarm und eine Visualisierungseinrichtung aus. Der Gelenkarm ist mit mindestens einem Gelenkarmabschnitt, einem Basisgelenk und einem Kopfgelenk ausgestattet. Das Basisgelenk kann einen oder mehrere Freiheitsgrade aufweisen. Entsprechendes gilt für das Kopfgelenk. Über das Basisgelenk ist der Gelenkarm an einer Gelenkarmbasis beweglich aufgenommen. Die Gelenkarmbasis ermöglicht die Anordnung der Ausgabevorrichtung in einem Raum, beispielsweise mittels eines fahrbaren oder in sonstiger Weise beweglichen Untersatzes oder mittels einer Befestigung an einer Wand, einer Decke oder einem Boden eines Raumes oder an einem Objekt im Raum. Die Gelenkarmbasis kann beispielsweise an einem Aufbau befestigt sein, der der Untersuchung mit einem 3D Endoskop oder mit einem 3D Mikroskop dient. Mittels des Kopfgelenks ist die Visualisierungseinrichtung beweglich an dem Gelenkarm angeordnet. Kern der Lösung der oben genannten Aufgabe ist, dass das Kopfgelenk ein erstes Kopfdrehgelenk, ein zweites Kopfdrehgelenk, ein drittes Kopfdrehgelenk und ein Kopplungsteil, welches das zweite mit dem dritten Kopfdrehgelenk verbindet, umfasst. Erfindungsgemäß stehen die Drehachsen des ersten und zweiten Kopfdrehgelenks senkrecht zueinander, wodurch eine intuitive Kipp- und Schwenkbewegung ermöglicht wird. Zusätzlich erlaubt das dritte Kopfdrehgelenk eine Drehbewegung der Visualisierungseinrichtung um ihre Längsachse. Durch diese spezifische Anordnung von drei entkoppelten Rotationsachsen direkt an der Visualisierungseinrichtung wird die in der Aufgabe geforderte vollständige, intuitive und mechanisch stabile dreidimensionale Feinausrichtung auf besonders vorteilhafte Weise erreicht.

Die Visualisierungseinrichtung umfasst ein Monitorgehäuse, in welchem ein linker Monitor und ein rechter Monitor angeordnet sind. Das Monitorgehäuse ist mit einem dem linken Monitor zugeordneten linken Sichtfenster ausgestattet, über welches der linke Monitor von außen einsehbar ist. Darüber hinaus ist das Monitorgehäuse mit einem dem rechten Monitor zugeordneten rechten Sichtfenster ausgestattet, über welches der rechte Monitor von außen einsehbar ist. Der Abstand zwischen dem linken Sichtfenster und dem rechten Sichtfenster entspricht einem typischen Augenabstand einer Person. Wenn eine Person ihren Kopf an das Monitorgehäuse anlegt, kann sie mit ihrem linken Auge durch das linke Sichtfenster auf den linken Monitor blicken und mit ihrem rechten Auge durch das rechte Sichtfenster auf den rechten Monitor blicken.

Zwischen dem linken Sichtfenster und dem linken Monitor können optische Komponenten, wie beispielsweise Linsen oder Prismen angeordnet sein.

Entsprechendes gilt für den Zwischenraum zwischen dem rechten Sichtfenster und dem rechten Monitor.

Der linke und der rechte Monitor können durch zwei voneinander räumliche getrennte und separate Monitore gebildet sein. Alternativ dazu können der linke und rechte Monitor Bereiche eines einzigen Monitors sein.

Die Visualisierungseinrichtung kann mit einem beliebigen Bildgebungssystem gekoppelt werden, das dreidimensionale Bilddaten eines Objektes erzeugt. Insbesondere kann die Visualisierungseinrichtung an ein 3D Videoendoskop oder an ein 3D Mikroskop gekoppelt sein. Das betreffende Bildgebungssystem erzeugt dabei Bilddaten für das linke Auge und für das rechte Auge. Diese Bilddaten werden über eine Schnittstelle so an die Visualisierungseinrichtung ausgegeben, dass die Bilddaten für das linke Auge auf dem linken Monitor und die Bilddaten für das rechte Auge auf dem rechten Monitor angezeigt werden. Die Person, welche das Objekt untersucht, erhält damit anhand der Visualisierungseinrichtung die Darstellung eines dreidimensionalen Bildes des Objektes. Da die Bilddaten für das linke Auge auf dem linken Monitor und für das rechte Auge auf dem rechten Monitor angezeigt werden und da der Benutzer durch die mit Sichtfenstern ausgestattete Visualisierungseinrichtung die Bildinformationen für das linke und rechte Auge getrennt voneinander angezeigt bekommt, kann auf das Tragen einer zusätzlichen Brille wie beispielsweise eine Polfilterbrille, Farbfilterbrille, Interferenzfilterbrille oder LCD-Shutterbrille verzichtet werden. Gegenüber bekannten Ausgabevorrichtungen, bei denen alle Bilddaten auf einem einzigen Monitor angezeigt werden, hat die erfindungsgemäße Ausgabevorrichtung damit den Vorteil, dass die Person keine zusätzliche Brille tragen muss, welche die Bilddaten für das rechte und linke Auge entsprechend filtert. Die Sicht der Person auf alle anderen Bereiche außerhalb des Monitors, wie beispielsweise auf den Einsatzort eines 3D Videoendoskops und ein eventuell zusammen mit diesem genutztes Instrument wird damit nicht hinsichtlich der Lichtintensität beeinträchtigt. Darüber hinaus bedeutet der Verzicht auf eine zusätzliche spezielle Brille einen erhöhten Komfort für die Person und erlaubt auch Brillenträgern, welche zur Korrektur einer Sehschwäche eine Brille tragen, die Visualisierungseinrichtung zu nutzen.

Der mit dem Basisgelenk und mit dem Kopfgelenk ausgestattete Gelenkarm ermöglicht die Einstellung und Ausrichtung der Visualisierungseinrichtung an ihrem Einsatzort. Eine Person kann mittels des Gelenkarms und der beiden Gelenke die Visualisierungseinrichtung so an ihre Körpergröße und an ihre Körperhaltung anpassen, dass sie bequem mit ihrem linken Auge durch das linke Sichtfenster und mit ihrem rechten Auge durch das rechte Sichtfenster blicken kann. Ferner kann sich die Ausgabevorrichtung auch automatisch der Körpergröße und der Körperhaltung der Person anpassen. Ist die Position der Visualisierungseinrichtung anhand des Gelenkarms eingestellt, kann der Gelenkarm fixiert werden. Damit ist gewährleistet, dass die Position der Visualisierungseinrichtung auch dann erhalten bleibt, wenn der Benutzer oder eine dritte Person die Visualisierungseinrichtung oder den Gelenkarm unabsichtlich berührt.

Die Person bekommt direkt auf ihrem linken Auge die auf dem linken Monitor dargestellte Bildinformation angezeigt und auf ihrem rechten Auge die auf dem rechten Monitor dargestellte Bildinformation. Ist das Bildgebungssystem ein 3D Videoendoskop, welches die Person zusammen mit einem Instrument manuell betätigt, so kann sie sowohl auf den Einsatzort des Endoskops und des Instruments blicken als auch durch die beiden Sichtfenster der Visualisierungseinrichtung auf den ersten und zweiten Monitor. Um beides einsehen zu können, muss die Person ihren Kopf allenfalls leicht drehen. Hierzu kann sie die Visualisierungseinrichtung so ausrichten, dass sie ihre Körperhaltung und ihre Blickrichtung allenfalls geringfügig ändern muss, um zwischen dem Einsatzort des Endeskops und des Instruments einerseits und dem linken und rechten Monitor andererseits zu wechseln. Dabei wird die Visualisierungseinrichtung von dem Gelenkarm gehalten, so dass die Person nach einmal erfolgter Einstellung der Position und Ausrichtung der Visualisierungseinrichtung die Hände frei hat, um sich am Einsatzort zu betätigen. Gegenüber einem einzigen an einer Wand oder sonstigen Halterung befestigten Monitor, auf dem alle Bilddaten für das rechte und linke Auge gemeinsam angezeigt werden, hat die erfindungsgemäße Ausgabevorrichtung damit den Vorteil, dass die Person ihre Körperhaltung und ihre Blickrichtung nicht oder allenfalls geringfügig ändern muss, um zwischen der Sicht auf einen Einsatzort eines Bildgebungssystems und der Sicht auf die Visualisierungseinrichtung zu wechseln.

Da die Visualisierungseinrichtung an dem Gelenkarm und nicht an dem Körper der Person befestigt ist, kann sich die Person jederzeit von der Visualisierungseinrichtung entfernen und/ oder die Visualisierungseinrichtung kann aus dem Blickfeld der Person entfernt werden. Es ist auch möglich, dass die Person nur für einen Augenblick an der Visualisierungseinrichtung vorbeischaut, um beispielsweise visuell zu prüfen, ob ein Instrument richtig positioniert ist. Nach Abschluss der Prüfung kann die Person unmittelbar wieder in die Visualisierungseinrichtung schauen. Gegenüber einem am Kopf der Person befestigen Sichtgerät hat die erfindungsgemäße Ausgabevorrichtung damit den Vorteil, dass die Person ihren Blick von dem linken und rechten Monitor lösen kann, ohne hierzu die Ausgabevorrichtung mit den Händen vom Kopf abnehmen zu müssen.

Der Gelenkarm und/ oder die Visualisierungseinrichtung können entweder rein manuell oder mit Unterstützung eines elektrischen Antriebs eingestellt werden. Zur rein manuellen Einstellung richtet die Person den Gelenkarm und/ oder die Visualisierungseinrichtung mit den Händen so aus, dass die Position für den jeweiligen Einsatz optimiert ist. Ist die 3D Ausgabevorrichtung mit einem elektrischen Antrieb ausgestattet, so kann die Person den Gelenkarm und/ oder die Visualisierungseinrichtung durch gezieltes Ein- und Ausschalten des Antriebs in die von ihm gewünschte Position bringen. Ist der elektrische Antrieb mit einer Steuerungseinrichtung und mit Sensoren oder Gebern ausgestattet, die die Person oder bestimmte Körperteile der Person erfassen, so kann die Einstellung der 3D Ausgabevorrichtung auch automatisch erfolgen. In diesem Fall muss die Person weder den Gelenkarm noch die Visualisierungseinrichtung oder gegebenenfalls eine Eingabeeinrichtung berühren, um die Einstellung der Ausgabevorrichtung vorzunehmen. Die Einstellung kann in diesem Fall berührungsfrei erfolgen.

Vorteilhafterweise wird die 3D Ausgabevorrichtung mit einer manuell betätigbaren Bildgebungseinrichtung kombiniert. Sie kann jedoch auch Bestandteil eines Chirurgieassistenzroboters sein.

Nach einer vorteilhaften Ausgestaltung der Erfindung weist das Monitorgehäuse eine Sichtfenster-Gehäusewand auf, in welcher das linke und rechte Sichtfenster angeordnet sind. Die Sichtfenster-Gehäusewand weist eine an die Kopfform einer Person angepasste Krümmung auf. Diese Form der Sichtfenster-Gehäusewand reduziert das Eindringen von Streulicht, wenn eine Person durch die beiden Sichtfenster blickt und dabei ihren Kopf an die Sichtfenster-Gehäusewand heranführt. Legt die Person ihren Kopf an die Sichtfenster-Gehäusewand an, wenn sie durch die beiden Sichtfenster auf die Monitore blickt, so unterstützt die Sichtfenster-Gehäusewand die Person dabei, ihren Kopf ruhig zu halten.

Nach einer weiteren vorteilhaften Ausgestaltung der Erfindung weist die Sichtfenster-Gehäusewand eine Nasenmulde zwischen dem linken und rechten Sichtfenster auf. Die Person kann damit ihre Augen unmittelbar an die beiden Sichtfenster heranführen, ohne dass ihre Nase hierbei stört.

Nach einer weiteren vorteilhaften Ausgestaltung der Erfindung ist das Monitorgehäuse zusätzlich mit mindestens einer Streulicht-Schutzblende ausgestattet, welche das Eindringen von Streulicht in das linke und rechte Sichtfenster verhindert oder zumindest reduziert. Die Streulicht-Schutzblende kann beispielsweise an der Sichtfenster-Gehäusewand angeordnet sein und über das Monitorgehäuse seitlich überstehen. Wenn eine Person ihren Kopf an die Sichtfenster-Gehäusewand anlegt und durch die beiden Sichtfenster blickt, legt sich die mindestens eine Streulicht-Schutzblende im Bereich der Stirn und der Schläfen an den Kopf der Person an. Es kann sich um eine durchgängige Streulicht-Schutzblende handeln, die an drei Seiten des Kopfes der Person für die Abschirmung von Streulicht sorgt. Es können jedoch auch drei separate Streulicht-Schutzblenden vorgesehen sein: eine für den Stirnbereich und zwei für den seitlichen Kopfbereich. Die Streulicht-Schutzblende kann aus einem weichen gummiartigen Material bestehen, so dass sie sich beim Anlegen des Kopfes verformen kann. Verletzungen der Person werden dadurch vermieden.

Nach einer weiteren vorteilhaften Ausgestaltung der Erfindung sind die beiden Sichtfenster mit Augenmuscheln ausgestattet, welche zusätzlich das Eindringen von Streulicht in das Monitorgehäuse verhindern, wenn eine Person durch das linke und rechte Sichtfenster blickt.

Nach einer weiteren vorteilhaften Ausgestaltung der Erfindung weist das Monitorgehäuse einen linken Gehäuseabschnitt auf, welcher mit dem linken Monitor und mit dem linken Sichtfenster ausgestattet ist. Ferner weist das Monitorgehäuse einen rechten Gehäuseabschnitt auf, welcher mit dem rechten Monitor und dem rechten Sichtfenster ausgestattet ist. Dabei ist der linke Gehäuseabschnitt von dem rechten Gehäuseabschnitt zumindest abschnittsweise abgetrennt, derart dass über das linke Sichtfenster nur der linke Monitor einsehbar ist und über das rechte Sichtfenster nur der rechte Monitor. Dadurch ist gewährleistet, dass eine Person, die durch die beiden Sichtfenster blickt, mit dem linken Auge nur die für das linke Auge vorgesehene Information angezeigt bekommt und mit dem rechten Auge nur die für das rechte Auge vorgesehene Information. Der linke und rechte Gehäuseabschnitt können beispielsweise durch einen Trennsteg im Monitorgehäuse voneinander abgetrennt sein. Darüber hinaus besteht die Möglichkeit, dass die Visualisierungseinrichtung als Binokular ausgeführt ist und der linke Gehäuseabschnitt von dem rechten Gehäuseabschnitt räumlich getrennt ist. Die beiden Gehäuseabschnitte sind in diesem Fall als zwei separate Gehäuseteile ausgeführt.

Nach einer weiteren vorteilhaften Ausgestaltung der Erfindung ist die Gelenkarmbasis höhenverstellbar ausgebildet, so dass die 23D Ausgabevorrichtung an die Größe einer Person angepasst werden kann.

Nach einer weiteren vorteilhaften Ausgestaltung der Erfindung weist die 3D Ausgabevorrichtung mindestens drei rotatorische Freiheitsgrade und einen translatorischen Freiheitsgrad auf, um die Visualisierungseinrichtung im Raum auszurichten.

Nach einer weiteren vorteilhaften Ausgestaltung der Erfindung umfasst das Kopfgelenk ein Kopfdrehgelenk, über das die Visualisierungseinrichtung um eine Drehachse frei beweglich an dem Gelenkarm gelagert ist. Dabei richtet sich die Visualisierungseinrichtung ihrer Gewichtskraft folgend stets horizontal aus. Ist die Visualisierungseinrichtung horizontal ausgerichtet, so verläuft eine Gerade, welche sich durch das Zentrum des linken Sichtfensters und durch das Zentrum des rechten Sichtfensters erstreckt, horizontal. Wird die Visualisierungseinrichtung durch äußere Krafteinwirkung aus der horizontalen Ausrichtung ausgelenkt, so kehrt die Visualisierungseinrichtung nach Wegfall dieser äußeren Kraft selbsttätig in die horizontale Ausrichtung zurück. Die horizontale Ausrichtung ist eine stabile Gleichgewichtslage der Visualisierungseinrichtung. Vorteilhafterweise ist die Visualisierungseinrichtung so mit dem Kopfgelenk verbunden, dass sich ihr Schwerpunkt unterhalb einer Drehachse des Drehgelenks befindet.

Nach einer weiteren vorteilhaften Ausgestaltung der Erfindung weist das Kopfdrehgelenk einen an dem Gelenkarm angeordneten Stift und eine an der Visualisierungseinrichtung angeordnete Lagerschale auf, welche den Stift umgreift. Alternativ kann auch der Stift an der Visualisierungseinrichtung angeordnet sein und die den Stift umgreifende Lagerschale an dem Gelenkarm.

Nach einer weiteren vorteilhaften Ausgestaltung der Erfindung weist das Kopfgelenk zusätzlich zu dem Drehgelenk mindestens ein weiteres Gelenk auf, mit dem die Neigung der Visualisierungseinrichtung bei horizontaler Ausrichtung der beiden Sichtfenster einstellbar ist. Durch die Einstellung der Neigung kann die Ausrichtung der Visualisierungseinrichtung an die Blickrichtung der Person angepasst werden. Vorteilhafterweise kann diese Einstellung arretiert werden, so dass sich die Einstellung nicht selbsttätig ändert.

Nach einer weiteren vorteilhaften Ausgestaltung der Erfindung weist der Gelenkarm mindestens zwei Gelenkarmabschnitte auf, die über ein Zwischengelenk miteinander beweglich verbunden sind. Das Zwischengelenk kann einen Freiheitsgrad oder mehrere Freiheitsgrade aufweisen, so dass die Gelenkarmabschnitte im Raum gedreht und verkippt werden können. Mit der Bewegung der Gelenkarmabschnitte wird die Visualisierungseinrichtung in ihrer Höhe und Ausrichtung an die Position der Person angepasst. Gegenüber einem Gelenkarm mit nur einem Gelenkarmabschnitt hat ein Gelenkarm mit zwei Gelenkarmabschnitten den Vorteil, dass mehr Einstellungsmöglichkeiten gegeben sind. Die beiden Gelenkarmabschnitte können unterschiedlich geformt und/ oder aus unterschiedlichen Materialien hergestellt sein. Zur Aufbewahrung oder zum Transport der 3D Ausgabevorrichtung kann der Gelenkarm so zusammengeklappt werden, dass die beiden Gelenkarmabschnitte aneinander liegen. In dieser Einstellung beansprucht der Gelenkarm besonders wenig Platz. Im Anwendungsfall kann beispielsweise ein erster Gelenkarmabschnitt vertikal nach oben ausgerichtet sein und ein zweiter Gelenkabschnitt in einem rechten Winkel zu dem ersten Gelenkarmabschnitt.

Nach einer weiteren vorteilhaften Ausgestaltung der Erfindung ist die Gelenkarmbasis fahrbar. Als fahrbare Gelenkarmbasis kann ein fahrbarer Untersatz wie ein Gestell mit Rollen oder ein Wagen vorgesehen sein. Dadurch ist es möglich, die 3D Ausgabevorrichtung schnell und einfach von einem Einsatzort zu einem anderen Einsatzort zu bewegen.

Nach einer weiteren vorteilhaften Ausgestaltung der Erfindung ist die Gelenkarmbasis mit einer Befestigungseinrichtung ausgestattet, mit welcher der Gelenkarm an einer Wand oder Decke oder einem Boden eines Raumes befestigbar ist. Darüber hinaus kann die Gelenkarmbasis mittels der Befestigungseinrichtung auch direkt an einem Tisch, insbesondere einem Operationstisch oder an einem Operationsroboter befestigt werden. Der Gelenkarm kann von der Befestigungseinrichtung lösbar sein.

Nach einer weiteren vorteilhaften Ausgestaltung der Erfindung ist sie mit mindestens einem Antrieb ausgestattet, über den die Ausrichtung des Gelenkarms und/ oder der Visualisierungseinrichtung einstellbar ist. Der Antrieb umfasst beispielsweise einen oder mehrere Elektromotoren.

Nach einer weiteren vorteilhaften Ausgestaltung ist der Antrieb mit einer Steuerungseinrichtung ausgestattet. Die Steuerungseinrichtung sorgt für die Einstellung des Gelenkarms und der Visualisierungseinrichtung in Abhängigkeit von vorgegebenen oder mittels Sensoren oder Gebern erfassten Größen. So kann beispielsweise die natürliche Körperhaltung und die Blickrichtung einer Person erfasst werden und die 3D Ausgabevorrichtung automatisch entsprechend ausgerichtet werden. Blickt die Person beispielsweise auf ein von ihr zu führendes Endoskop, so wird diese Blickrichtung beim Betrachten des 3D-Bildes beibehalten. Der Antrieb kann auch mittels eines Programms der Steuereinrichtung folgen, so dass sich der Gelenkarm und/ oder die Visualisierungseinrichtung nach bestimmten Vorgaben positionieren und ausrichten. Der Gelenkarm und/ oder die Visualisierungseinrichtung werden dabei durch den Antrieb bewegt, so dass eine Person sie beim Ausrichten nicht berühren muss.

Nach einer weiteren vorteilhaften Ausgestaltung der Erfindung ist die Steuerungseinrichtung mit mindestens einem Sensor ausgestattet, welcher Messdaten erfasst und diese an die Steuereinrichtung übermittelt. Es können beispielsweise optische Sensoren eingesetzt werden. Diese nehmen beispielsweise Markierungen wahr, welche an der Person oder im Raum vorgesehen sind. Auch in diesem Fall kann die Bewegung des Gelenkarms und/ oder der Visualisierungseinrichtung erfolgen, ohne dass eine Person sie hierzu berühren muss.

Nach einer weiteren vorteilhaften Ausgestaltung umfasst die Steuerungseinrichtung eine Sprachsteuerung. Über ein Mikrophon werden Sprachbefehle einer Person aufgenommen und in entsprechende Steuersignale des Antriebs umgesetzt.

Nach einer weiteren vorteilhaften Ausgestaltung umfasst die Steuerungseinrichtung eine Bewegungssteuerung. Bewegungen einer Person werden erfasst und ausgewertet, um den Antrieb zu steuern. Zur Erfassung der Bewegungen werden vorzugsweise Sensoren eingesetzt.

Nach einer weiteren vorteilhaften Ausgestaltung der Erfindung ist die Steuerungseinrichtung mit einer Eingabeeinrichtung ausgestattet, die eine Person manuell betätigen kann. Die Eingabeeinrichtung kann unter anderem mit Steuerungstasten und einem Steuerungshebel ausgestattet sein. In diesem Fall erfolgt die Einstellung des Gelenkarmes und der Visualisierungseinrichtung durch Eingabe von entsprechenden Befehlen einer Person.

Die Einstellung des Gelenkarms und der Visualisierungseinrichtung kann auch rein manuell durch Krafteinwirkung erfolgen, die eine Person manuell aufbringt. Eine manuelle Steuerung kann auch dadurch erfolgen, dass die Person den Gelenkarm und/ oder die Visualisierungseinrichtung direkt berührt und führt. Beispielsweise indem er seinen Kopf an das Monitorgehäuse anlegt und dieses nach vorne schiebt.

Nach einer weiteren vorteilhaften Ausgestaltung der Erfindung ist die 3D Ausgabevorrichtung mit einer Feststelleinrichtung ausgestattet, mit der eine eingestellte Ausrichtung des Gelenkarms und/ oder der Visualisierungseinrichtung feststellbar ist. Mittels der Feststelleinrichtung können das Basisgelenk, das Kopfgelenk und gegebenenfalls ein Zwischengelenk arretiert werden. Die Gelenke können einzeln oder zusammen arretiert werden. Gleiches gilt für den umkehrten Fall, also dem beweglichen Zustand der Gelenke. Die Gelenke können beispielsweise mittels Druckluft arretiert werden. Die Feststellen und das Lösen können durch einen Schalter oder Taster ausgelöst werden, der an dem Monitorgehäuse der Visualisierungseinrichtung angeordnet ist und manuell betätigt werden kann. Alternativ kann ein Schalter oder Taster auf dem Boden vorgesehen sein und mit dem Fuß betätigt werden.

Nach einer weiteren vorteilhaften Ausgestaltung der Erfindung ist der Gelenkarm mit einem Griff ausgestattet, mit dem die Ausrichtung des Gelenkarms manuell einstellbar ist. Griffe können an jedem Gelenkarmabschnitt vorgesehen sein. Auch die Visualisierungseinrichtung kann mit einem Griff ausgestattet sein. In sterilen Umgebungen ist es entscheidend, dass nur bestimmte Flächen berührt werden. Ein Griff stellt genau dies sicher. Die übrigen Teile des Gelenkarmes und der Visualisierungseinrichtung bleiben steril.

Nach einer weiteren vorteilhaften Ausgestaltung der Erfindung ist der Gelenkarm und/ oder die Visualisierungseinrichtung mit mindestens einem austauschbaren Hygiene-Aufsatz ausgestattet. Der Hygiene-Aufsatz besteht beispielsweise aus einem Silikonmaterial. Die Hygiene-Aufsätze sind insbesondere an den Stellen vorgesehen, die berührt werden, also beispielsweise an Griffen, Steuerhebeln und Auflageflächen. Um die Keimfreiheit zu gewährleisten, werden die Hygiene-Aufsätze nach jedem Einsatz gereinigt, desinfiziert, sterilisiert oder ausgetauscht. Für diesen Zweck sind beispielsweise leicht klebende oder aufsteckbare starre oder flexible Silikonkomponenten vorgesehen. Diese können auch mit den Auflageflächen verschraubt sein. Darüber hinaus geben Silikonkomponenten auf Druck nach und passen sich den Konturen der Person an, so dass ein zusätzlicher Komfort geschaffen wird. Die weiteren Teile der Ausgabevorrichtung sind regelmäßig mit einer sterilen Hülle oder einer sterilen Membran umhüllt und werden nicht berührt.

Nach einer weiteren vorteilhaften Ausgestaltung der Erfindung ist die 3D Ausgabevorrichtung mit einem Bildgebungssystem ausgestattet. Im medizinischen Bereich werden als Bildgebungssysteme insbesondere Endoskope, Mikroskope, Laparoskope, Ultraschallgeräte, Röntgengeräte oder einem anderen medizinischen Bildgebungssystem eingesetzt. Das Bildgebungssystem nimmt über einen linken optischen Kanal Bilder für das linke Auge einer Person auf und mit einem rechten optischen Kanal Bilder für das rechte Auge einer Person. Die Bilder werden an die Visualisierungseinrichtung übermittelt. Hierfür können im Inneren des Gelenkarms in Richtung der Visualisierungseinrichtung verlaufende Versorgungsleitungen vorgesehen sein. Auch können die aufgenommenen Bilder über Schnittstellen, wie drahtlose Schnittstellen an die Visualisierungseinrichtung übermittelt werden. Letzteres bietet sich insbesondere dann an, wenn die aufgenommenen Bilder an eine Visualisierungseinrichtung übermittelt werden sollen, die sich nicht im gleichen Raum wie das untersuchte Objekt befindet oder wenn diese an mehrere Visualisierungseinrichtungen übermittelt werden sollen.

Weitere Vorteile und vorteilhafte Ausgestaltungen der Erfindung sind der nachfolgenden Beschreibung, der Zeichnung und den Ansprüchen entnehmbar.

### Zeichnung

In der Zeichnung ist ein Ausführungsbeispiele des Erfindungsgegenstandes dargestellt. Es zeigen:
- Figur 1: 3D Ausgabevorrichtung in einer perspektivischen Ansicht,
- Figur 2: Visualisierungseinrichtung und ein Abschnitt des Gelenkarms der 3D Ausgabevorrichtung gemäß Figur 1,
- Figur 3: 3D Ausgabevorrichtung gemäß Figur 1 positioniert an einem Tisch in einer perspektivischen Ansicht,
- Figur 4: 3D Ausgabevorrichtung gemäß Figur 1 mit einer zur Seite geschwenkten Visualisierungseinrichtung in einer Ansicht von der Seite,
- Figur 5: 3D Ausgabevorrichtung gemäß Figur 4 in einer Ansicht von oben,
- Figur 6: 3D Ausgabevorrichtung gemäß Figur 1 mit einer gedrehten Visualisierungseinrichtung in einer Ansicht von der Seite,
- Figur 7: 3D Ausgabevorrichtung gemäß Figur 6 in einer Ansicht von oben,
- Figur 8: 3D Ausgabevorrichtung gemäß Figur 1, wobei ein Gelenkarmabschnitt des Gelenkarms geschwenkt ist, in einer Ansicht von der Seite,
- Figur 9: 3D Ausgabevorrichtung gemäß Figur 8 in einer Ansicht von oben,
- Figur 10: 3D Ausgabevorrichtung gemäß Figur 1 mit nach oben gekippter Visualisierungseinrichtung in einer Ansicht von der Seite,
- Figur 11: 3D Ausgabevorrichtung gemäß Figur 10 in einer Ansicht von oben,
- Figur 12: Visualisierungseinrichtung der 3D Ausgabevorrichtung gemäß Figur 1 in einer perspektivischen Ansicht,
- Figur 13: Visualisierungseinrichtung der 3D Ausgabevorrichtung gemäß Figur 1 in einer Ansicht von vorne,
- Figur 14: Visualisierungseinrichtung der 3D Ausgabevorrichtung gemäß Figur 1 in einer Ansicht von der Seite,
- Figur 15: Visualisierungseinrichtung der 3D Ausgabevorrichtung gemäß Figur 1 in einer Ansicht von oben,
- Figur 16: 3D-Ausgabevorrichtung gemäß Figur 1 mit einer an einem Tisch sitzenden Person in einer Ansicht von der Seite;
- Figur 17: 3D-Ausgabevorrichtung gemäß Figur 1 mit einer an einem Tisch stehenden Person in einer Ansicht von der Seite.

### Beschreibung des Ausführungsbeispiels

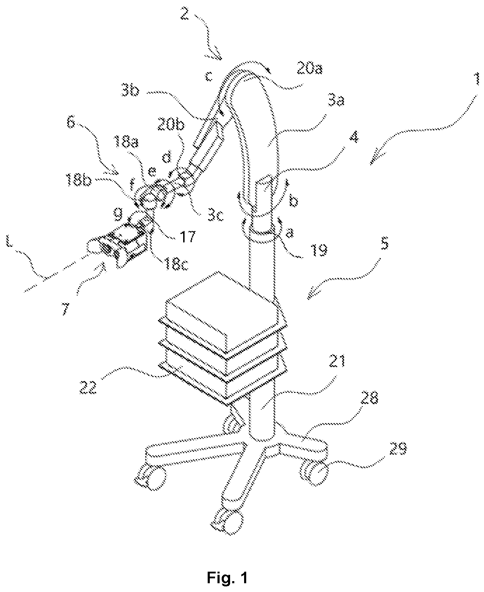

Figur 1 zeigt eine 3D Ausgabevorrichtung 1 mit einem Gelenkarm 2 und einer an dem Gelenkarm 2 beweglich angeordneten Visualisierungseinrichtung 7 in einer perspektivischen Ansicht. Der Gelenkarm 2 ist an einer Gelenkarmbasis 5 beweglich aufgenommen. Als Gelenkarmbasis 5 ist ein fahrbarer Gerätewagen mit feststellbaren Doppellenkrollen 29 vorgesehen. Die Gelenkarmbasis 5 weist eine Säule 21 mit einem Standfuß 28 und mehreren an dem Standfuß 28 angeordneten Doppellenkrollen 29 auf, von denen zumindest eine feststellbar ist. An der Säule 21 der Gelenkarmbasis 5 ist ein Schaft 19 höhenverstellbar und um seine Längsachse drehbar aufgenommen. Dieser Schaft ist Bestandteil der Gelenkarmbasis 5. Die Drehbewegung des Schaftes 19 relativ zu der Säule 21 ist mit dem Buchstaben a gekennzeichnet. Wird der Schaft 19 nach oben oder unten bewegt, so bewegt sich der Gelenkarm 2 entsprechend nach oben oder nach unten. So kann der Gelenkarm 2 an die Körpergröße eines Benutzers angepasst werden. An der Säule 21 sind mehre Geräteplatten 22 zur Ablage von Geräten und Instrumenten, insbesondere von Bildgebungsinstrumenten angeordnet. Der Gelenkarm 2 umfasst drei Gelenkarmabschnitte, nämlich den ersten Gelenkarmabschnitt 3a, den zweiten Gelenkarmabschnitt 3b und den dritten Gelenkarmabschnitt 3c.

Ein Ende des ersten Gelenkarmabschnittes 3a ist mit der Gelenkarmbasis 5 über ein Basisgelenk beweglich verbunden, welches in Figur 1 an der mit der Bezugszahl 4 gekennzeichneten Position angeordnet ist. Als Basisgelenk ist ein Drehgelenk mit einem Freiheitsgrad vorgesehen. Hierzu weist der Schaft 19 an seinem dem ersten Gelenkarmabschnitt 3a zugewandten Ende eine gabelförmige Aufnahme auf, in welcher der erste Gelenkarmabschnitt 3a um eine in der Zeichnung nicht erkennbare Achse drehbar aufgenommen ist. Diese Achse bildet eine zweite Drehachse des Gelenkarmes 2. Die Drehbewegung des Basisgelenks ist mit dem Buchstaben b gekennzeichnet.

Das der Gelenkarmbasis abgewandte Ende des ersten Gelenkarmabschnittes 3a ist über ein erstes Zwischendrehgelenk mit einem Ende eines zweiten Gelenkarmabschnittes 3b beweglich verbunden. Die Position des ersten Zwischendrehgelenkes ist durch die Bezugszahl 20a gekennzeichnet. Das erste Zwischendrehgelenk bildet eine dritte Drehachse des Gelenkarms 2. Die Drehbewegung des ersten Zwischendrehgelenkes ist mit dem Buchstaben c gekennzeichnet. Die geometrischen Drehachsen des Basisgelenks und des ersten Zwischendrehgelenkes sind parallel. Daraus folgt, dass sich der erste Gelenkarmabschnitt 3a und der zweite Gelenkarmabschnitt 3b in einer gemeinsamen Ebene bewegen.

Das dem ersten Gelenkarmabschnittes 3a abgewandte Ende des zweiten Gelenkarmabschnittes 3b ist über ein zweites Zwischendrehgelenk mit einem Ende eines dritten Gelenkarmabschnittes 3c beweglich verbunden. Die Position des zweiten Zwischendrehgelenks ist durch die Bezugszahl 20b gekennzeichnet. Das zweite Zwischendrehgelenk bildet eine vierte Drehachse des Gelenkarms 2. Die Drehbewegung des zweiten Zwischendrehgelenkes ist mit dem Buchstaben d gekennzeichnet. Die geometrische Drehachse des zweiten Zwischendrehgelenks verläuft senkrecht zu den geometrischen Drehachsen des Basisdrehgelenks und des ersten Zwischendrehgelenks. Eine Bewegung des dritten Gelenkarmabschnitts 3c relativ zu dem zweiten Gelenkarmabschnitt 3b um zweiten Zwischendrehgelenk führt nicht zu einer Veränderung des Winkels zwischen diesen beiden Gelenkarmabschnitten, sondern zu einer Rotation des dritten Gelenkarmabschnitts 3c um seine Längsachse.

Bei dem ersten Zwischendrehgelenk und dem zweiten Zwischendrehgelenk handelt es sich entsprechend dem Basisgelenk um Drehgelenke mit einem Freiheitsgrad.

Das dem zweiten Gelenkarmabschnitt 3b abgewandte Ende des dritten Gelenkarmabschnitts 3c ist über ein Kopfgelenk mit der Visualisierungseinrichtung 7 verbunden. Die Position des Kopfgelenkes ist durch die Bezugszahl 6 gekennzeichnet. Das Kopfgelenk umfasst ein erstes Kopfdrehgelenk 18a, ein zweites Kopfdrehgelenk 18b, ein drittes Kopfdrehgelenk 18c und ein Kopplungsteil 17, welches das zweite Kopfdrehgelenk 18b mit dem dritten Kopfdrehgelenk 18c verbindet. Das erste, zweite und dritte Kopfdrehgelenk 18a, 18b, 18c bildet eine fünfte, sechste und siebte Drehachse des Gelenkarms 2. Eine durch das erste Kopfdrehgelenk 18a ermöglichte Drehbewegung ist durch den Buchstaben e gekennzeichnet. Ein durch das zweite Kopfdrehgelenk 18b ermöglichte Drehbewegung ist durch den Buchstaben f gekennzeichnet. Eine durch das dritte Kopfdrehgelenk 18c ermöglichte Drehbewegung ist durch den Buchstaben g gekennzeichnet. Die geometrischen Drehachsen des ersten Kopfdrehgelenkes 18a und des zweiten Kopfdrehgelenkes 18b stehen senkrecht zueinander. Dabei verläuft die geometrische Drehachse des ersten Kopfdrehgelenks 18a horizontal und die geometrische Drehachse des zweiten Kopfdrehgelenks 18b vertikal. Das erste Kopfdrehgelenk 18a und zweite Kopfdrehgelenk 18b weisen jeweils einen Freiheitsgrad auf.

Das Kopplungsteil 17 weist an seinem ersten Ende das zweite Kopfdrehgelenk 18b auf und an seinem dem ersten Ende gegenüberliegenden zweiten Ende das dritte Kopfdrehgelenk 18c. Das dritte Kopfdrehgelenk 18c ermöglich eine Drehbewegung der Visualisierungseinrichtung 7 um die Längsachse L der vorliegend länglich ausgebildeten Visualisierungseinrichtung 7. Die Längsachse L der Visualisierungseinrichtung 7 ist durch eine gestrichelte Linie dargestellt.

Insgesamt weist der Gelenkarm 2 mit dem Drehgelenk der Gelenkarmbasis 5, dem Basisgelenk, dem ersten Kopfdrehgelenk 18a, dem zweiten Kopfdrehgelenk 18b, dem dritten Kopfdrehgelenk 18c, dem ersten Zwischendrehgelenk und dem zweiten Zwischendrehgelenk sieben Drehachsen auf, die eine Einstellung der Visualisierungseinrichtung 7 ermöglichen. Dank des in der Säule 21 höhenverstellbar aufgenommenen Schaftes 19 ist darüber hinaus ein linearer Freiheitsgrad gegeben.

Die Gelenkarmabschnitte 3a, 3b, 3c des Gelenkarmes 2 und die Visualisierungseinrichtung 7 werden durch manuelle Kraftübertragung einer Person oder unterstützt durch einen Antrieb in Bewegung versetzt und ausgerichtet. Ein derartiger Antrieb ist in der Zeichnung nicht dargestellt. Dabei werden alle oder nur ein Teil der Gelenke des Gelenkarmes 2 bewegt, um den Gelenkarm 2 und die Visualisierungseinrichtung 7 von einer ersten Position in eine zweite Position zu bewegen. In einer eingestellten Position können die Gelenke des Gelenkarmes 2 mittels einer in Figur 1 nicht dargestellten Feststelleinrichtung arretiert werden.

Wird der erste Gelenkarmabschnitt 3a durch das Basisgelenk bewegt, so bildet sich zwischen der Säule 21 und dem ersten Gelenkarmabschnitt 3a ein spitzer oder stumpfer Winkel aus. Wird das Basisgelenk des Gelenkarmes 2 durch Druckluft arretiert, so bleibt der erste Gelenkarmabschnitt 3a in einer festen Position, bis die Arretierung des Basisgelenks gelöst und der erste Gelenkarmabschnitt 3a bewegt wird. Dies gilt entsprechend für die weiteren Gelenke und Gelenkarmabschnitte 3b, 3c des Gelenkarmes 2.

Wird das erste Zwischendrehgelenk durch Druckluft arretiert, das Basisgelenk jedoch nicht, so bleibt der Winkel, den der erste Gelenkarmabschnitt 3a und der zweite Gelenkarmabschnitt 3b miteinander einschließen, gleich, auch dann, wenn der erste Gelenkarmabschnitt 3a durch das Basisgelenk bewegt wird. In diesem Fall wird der zweite Gelenkarmabschnitt 3b durch das Basisgelenk bewegt. Werden alle Gelenke des Gelenkarmes 2 arretiert, so bewegen sich der Gelenkarm 2 und die Visualisierungseinrichtung 7 nicht mehr, selbst dann nicht, wenn der Gelenkarm 2 oder die Visualisierungseinrichtung 7 durch einen Benutzer unbeabsichtigt berührt werden.

Durch die sieben Drehachsen des Gelenkarmes 2 ist eine Ausrichtung der Visualisierungseinrichtung 7 auch dann schnell und einfach möglich, wenn komplexe Bewegungen des Gelenkarmes 2 ausgeführt werden sollen.

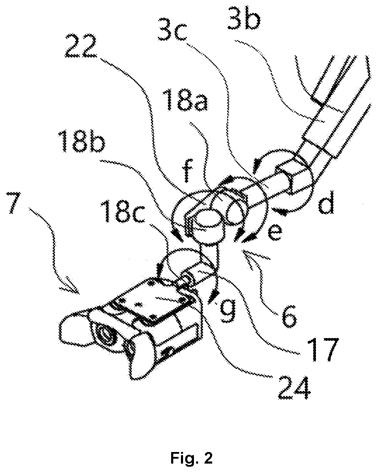

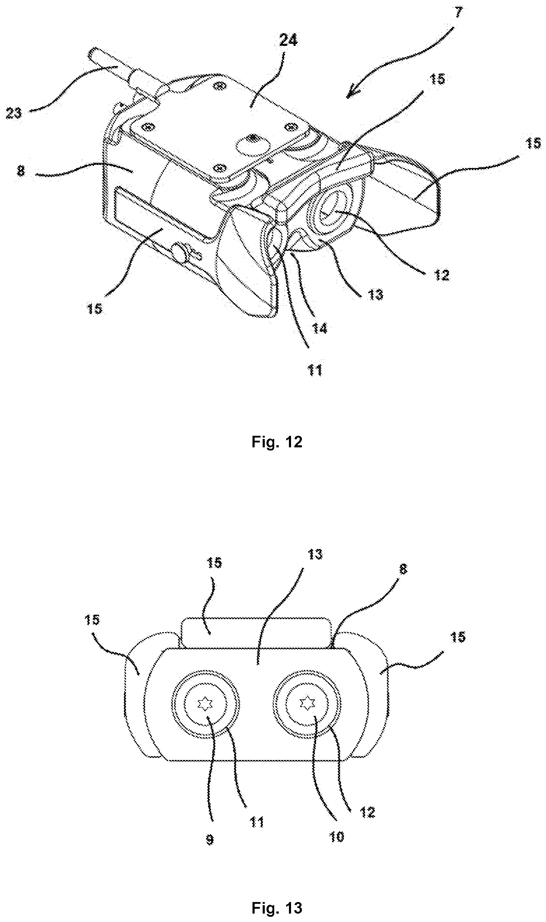

Figur 2 zeigt eine Detailansicht des Kopfgelenks 6. Das Kopfgelenk 6 umfasst das erste Kopfdrehgelenk 18a, das zweite Kopfdrehgelenk 18b und das dritte Kopfdrehgelenk 18c. Das erste Kopfdrehgelenk 18a ist über das Verbindungsstück 22 mit dem zweiten Kopfdrehgelenk 18b fest verbunden. Eine Drehung um die Drehachse des ersten Kopfdrehgelenk 18a führt dazu, dass das Verbindungsstück 22 und das zweite Kopfdrehgelenk 18b nach oben oder unten bewegt werden. Das zweite Kopfdrehgelenk 18b nimmt ein Ende des Kopplungsteils 17 auf, so dass sich dieses mittels des zweiten Kopfdrehgelenks 18b drehen kann. Das dem zweiten Kopfdrehgelenk 18b abgewandte Ende des Kopplungsteils 17 nimmt das dritte Kopfdrehgelenk 18c auf, über das es beweglich mit der Visualisierungseinrichtung 7 verbunden ist. Hierzu ist an der Visualisierungseinrichtung 7 eine fest verschraubte Platte 24 vorgesehen. Die Details hierzu sind in Figur 12 dargestellt. Um die Visualisierungseinrichtung 7 dreidimensional im Raum auszurichten, können die Gelenkarmabschnitte 3a, 3b, 3c sowie die Visualisierungseinrichtung um die verschiedenen Drehgelenke entsprechend den Pfeilen b, c, d, e, f und g bewegt werden.

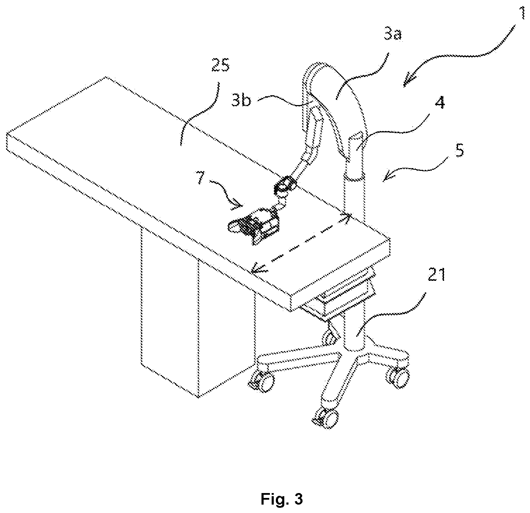

Figur 3 zeigt die 3D Ausgabevorrichtung 1 gemäß Figur 1, angeordnet an einem Tisch 25. Die Gelenkarmbasis 5 ist auf dem Boden angeordnet, so dass Säule 21 der Gelenkarmbasis teilweise unterhalb und teilweise oberhalb des Tisches 25 angeordnet ist. Die Visualisierungseinrichtung 7 ist oberhalb des Tisches 25 angeordnet. Wird der erste Gelenkarmabschnitt 3a bewegt, so sorgt das Basisgelenk, welches an der mit der Bezugszahl 4 gekennzeichneten Position angeordneten ist, dafür, dass sich der erste Gelenkarmabschnitt 3a der Oberfläche des Tisches 25 annähert oder sich von dieser entfernt. Wird der zweite Gelenkarmabschnitt 3b relativ zu dem ersten Gelenkarmabschnitt 3a bewegt, so bewegt sich die Visualisierungseinrichtung 7 in Richtung des gestrichelten Doppelpfeiles. Vorliegend ist die Oberseite der Visualisierungseinrichtung 7 horizontal zur Oberfläche des Tisches 25 in einem Abstand zu diesem angeordnet.

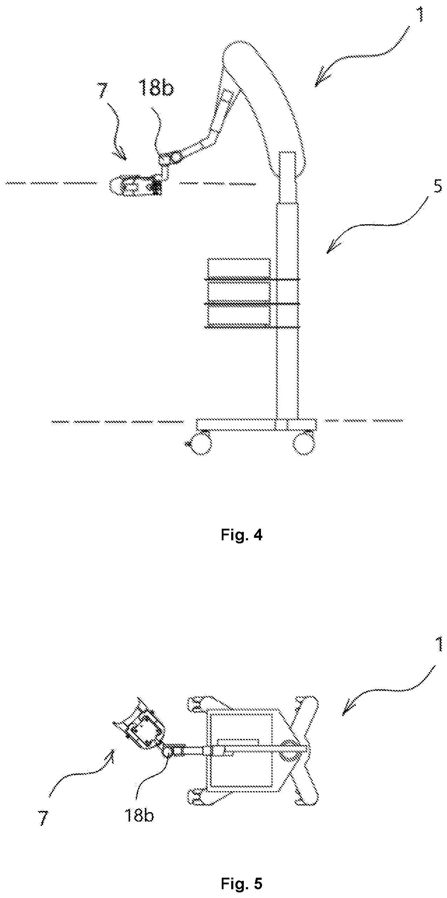

Die Figuren 4 und 5 zeigen die 3D-Ausgabevorrichtung 1 gemäß Figur 1 mit einer horizontal ausgerichteten und zu einer Seite geschwenkten Visualisierungseinrichtung 7 in einer Ansicht von der Seite und von oben. Die horizontale Ausrichtung der Visualisierungseinrichtung 7 wird durch zwei gestrichelte Linien angedeutet, von denen die eine die Ausrichtung des Bodens darstellt, auf dem die Gelenkarmbasis 5 angeordnet ist und die andere die Ausrichtung der Visualisierungseinrichtung 7 darstellt. Die seitliche Schwenkbewegung der Visualisierungseinrichtung 7 wird durch eine Bewegung des Kopplungsteils 17 um das erste Kopfdrehgelenk 18b bewirkt. Die Visualisierungseinrichtung 7 wird insbesondere dann zur Seite geschwenkt, wenn diese für einen Augenblick aus dem Blickfeld einer Person entfernt werden soll.

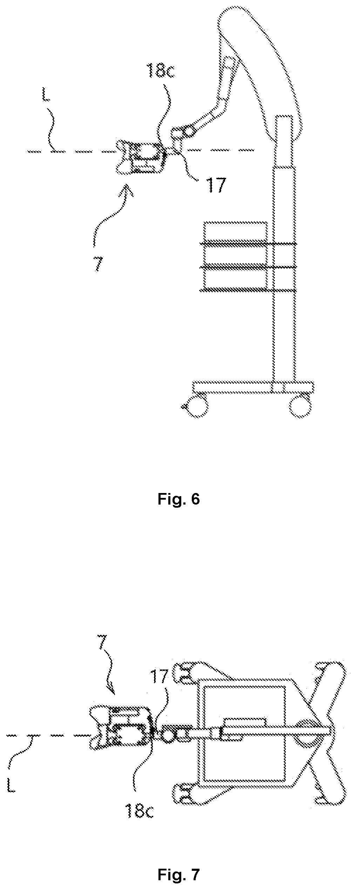

Die Figuren 6 und 7 zeigen die 3D-Ausgabevorrichtung gemäß Figur 1 mit einer um die Drehachse des dritten Kopfdrehgelenkes 18c gedrehten Visualisierungseinrichtung 7 in einer Ansicht von der Seite von oben. Die Drehachse des dritten Kopfdrehgelenks 18c ist koaxial zur Längsachse L der Visualisierungseinrichtung 7 angeordnet. Das dritte Kopfdrehgelenk 18c ist an dem der Visualisierungseinrichtung 7 zugewandten Ende des Kopplungsteils 17 angeordnet. Im Unterschied zu den vorhergehenden Figuren ist die Oberseite der Visualisierungseinrichtung 7 nicht mehr horizontal zum Boden ausgerichtet. Wird das dritte Kopfdrehgelenk 18c arretiert, so verbleibt die Visualisierungseinrichtung 7 in dieser um die Drehachse des dritten Kopfdrehgelenkes 18c gedrehten Position. Wird die Arretierung des dritten Kopfdrehgelenks 18c gelöst, so dreht sich die Oberseite der Visualisierungseinrichtung 7 selbstständig, ihrer Schwerkraft folgend zurück in ihre horizontal zum Boden ausgerichtete Gleichgewichtslage. Eine um die Drehachse des dritten Kopfdrehgelenkes 18c gedrehte Ausrichtung der Visualisierungseinrichtung 7 ist insbesondere dann vorteilhaft, wenn ein in der Figur 6 nicht dargestelltes Bildgebungssystem in einem zu untersuchenden Objekt gedreht wird und der Benutzer der Drehung des Bildgebungssystem mit seinem Blick folgen möchte.

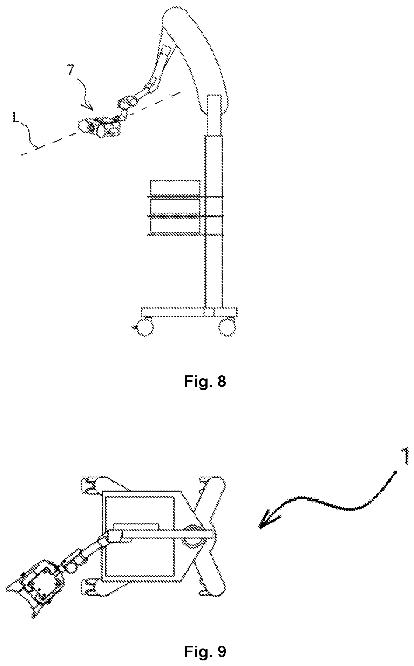

In den Figuren 8 und 9 ist ein Ausführungsbeispiel dargestellt, bei dem der zweite Gelenkarmabschnitt 3b an dem ersten Gelenkarmabschnitt 3a zusätzlich um eine vertikale Drehachse drehbar aufgenommen ist oder der zweite Gelenkarmabschnitt 3b selbst ein zusätzliches Drehgelenk aufweist. Figur 8 und 9 zeigen eine Drehung des zweiten Gelenkarmabschnitts 3b relativ zu dem ersten Gelenkarmabschnitts 3a um ein derartiges Drehgelenk.

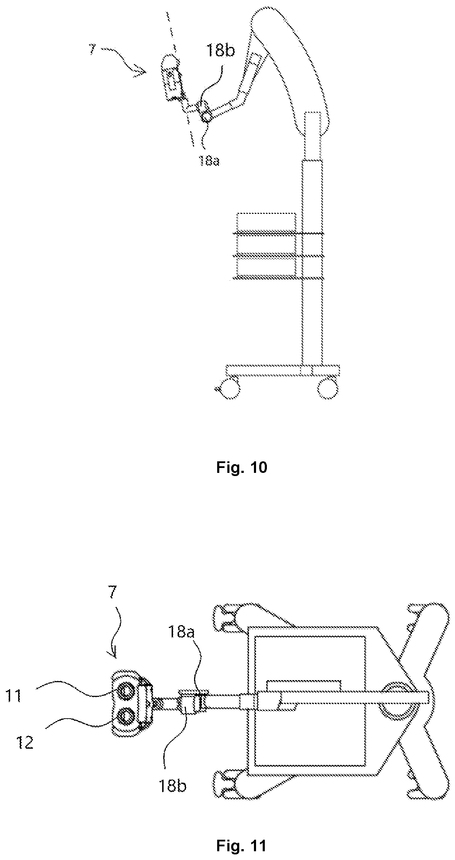

Die Figuren 10 und 11 zeigen die 3D-Ausgabevorrichtung gemäß Figur 1 mit einer um die Drehachse des ersten Kopfdrehgelenkes 18a gedrehten und dadurch nach oben gekippten Visualisierungseinrichtung 7 in einer Ansicht von der Seite und von oben. Durch eine Drehung der Visualisierungseinrichtung 7 um die Drehachse des ersten Kopfdrehgelenkes 18a bewegt sich das zweite Kopfdrehgelenk 18b nach oben, so dass das zweite Kopfdrehgelenk 18b oberhalb des ersten Kopfdrehgelenks 18a angeordnet ist. Die gekippte Anordnung der Visualisierungseinrichtung 7 ist insbesondere dann von Vorteil, wenn eine Person aufrecht steht und von oben in die linken und rechten Sichtfenster 11, 12 der Visualisierungseinrichtung 7 auf ein zu untersuchendes Objekt blickt.

Figur 12 zeigt die Visualisierungseinrichtung 7 in einer perspektivischen Ansicht. Gut zu erkennen ist, dass die Platte 24 über vier Schrauben an der Oberseite der Visualisierungseinrichtung 7 befestigt ist. Die Platte schließt im Wesentlichen mit den seitlichen Rändern der Oberseite der Visualisierungseinrichtung 7 ab. An dem rückseitigen Ende der Platte ist der Stift 23 vorgesehen, welcher von dem in Figur 12 nicht dargestellten Kopplungsteil 17 drehbar aufgenommen wird. An dem Monitorgehäuse 8 ist eine verstellbar und lösbar angeordnete obere Streulichtblende 15 angeordnet, die verhindert, dass Licht von oben in den Bereich der Sichtfenster-Gehäusewand 13 einfällt. Darüber hinaus sind an den Seitenflächen des Monitorgehäuses 8 zwei weitere seitliche Streulichtblenden 15 vorgesehen, die verhindern, dass Licht von der Seite in den Bereich der Sichtfenster-Gehäusewand 13 einstrahlt. Die Streulichtblenden 15 sind aus einem verformbaren Silikonmaterial ausgebildet. Die Sichtfenster-Gehäusewand 13 ist mit einer Nasenmulde 14 ausgestattet, welche sich zwischen dem linken und rechten Sichtfenster 11, 12 befindet. Eine Person kann ihre Stirn und ihre Nase an die Sichtfenster-Gehäusewand 13 anlegen und muss keine spezielle Brille tragen. Ist die Person Brillenträger, muss sie ihre Brille beim Blick durch das linke und rechte Sichtfenster 11, 12 nicht ablegen. Auf diese Weise kann eine Person gleichzeitig das vor ihr liegendes Objekt und das 3D-Bild des Objektes scharf sehen.

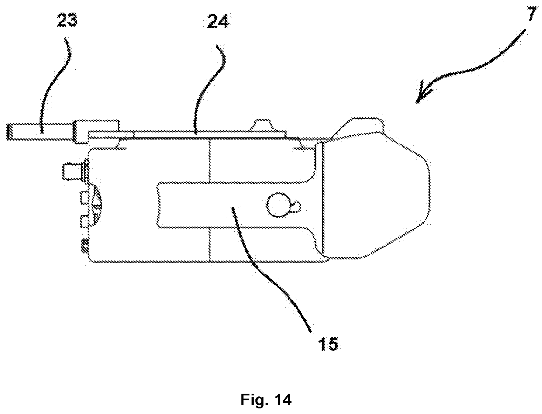

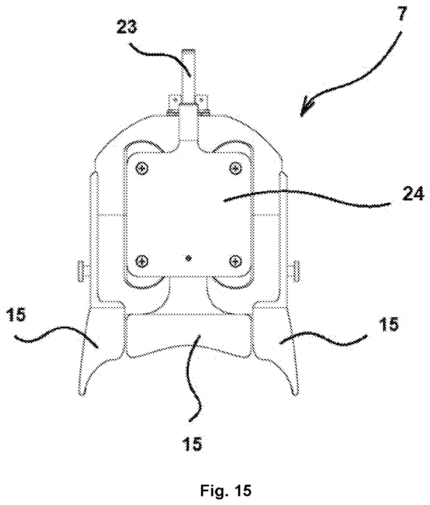

Die Figuren 13, 14 und 15 zeigen die Visualisierungseinrichtung 7 gemäß Figur 1 in einer Ansicht von vorn, von der Seite und von oben. In Figur 13 ist gut zu erkennen, dass auf den linken und rechten Monitoren 9, 10 eine Struktur des untersuchten Objektes angezeigt wird. Die Struktur ist vorliegend sternförmig ausgebildet. Die linken rechten Monitore 9, 10 sind innerhalb des Monitorgehäuses 8 in Verlängerung zu dem rund ausgebildeten linken Sichtfernster 11 und zu dem ebenfalls rund ausgebildeten rechten Sichtfenster 11, 12 angeordnet. Die Sichtfenstergehäusewand 13 wird von den Streulichtblenden 15 begrenzt. In Figur 13 ist die Nasenmulde nicht dargestellt. Figur 14 zeigt, dass die Visualisierungseinrichtung 7 mit ihrer Oberseite an der Platte 24 befestigt ist. Dies ermöglicht ein schnelles und einfaches Montieren der Visualisierungseinrichtung 7. Über den Stift 23 der Platte 24 wird die Visualisierungseinrichtung 7 von dem in Figur 14 nicht dargestellten Kopplungsteil 17 aufgenommen. Die Streulichtblenden 15 sind in Längsrichtung der Visualisierungseinrichtung 7 bewegbar. Figur 15 zeigt, dass die Streulichtblenden 15 ergonomisch der Kopfform eines Benutzers angepasst sind und über die Sichtfenstergehäusewand hinausragen, so dass auch ein Benutzer, der eine Brille trägt und daher einen gewissen Abstand zu den linken und rechten Sichtfenstern einhält, nicht von Umgebungslicht geblendet wird.

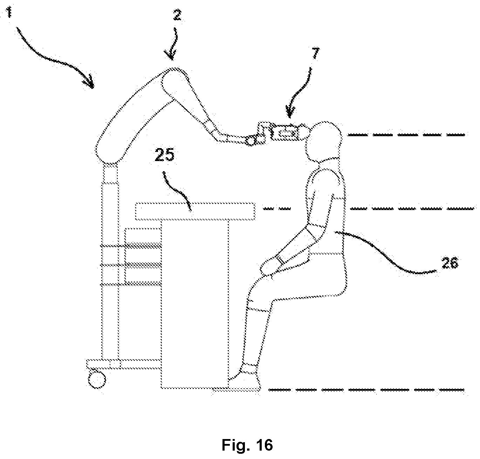

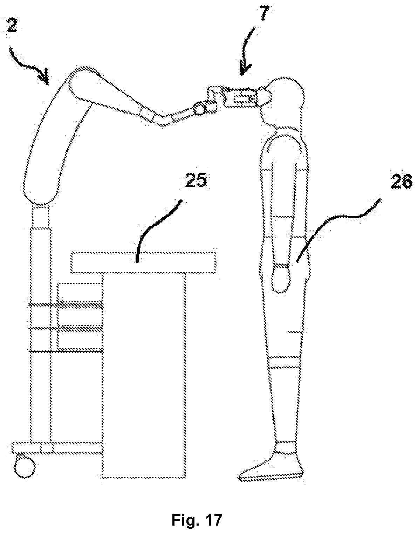

Figur 16 zeigt die 3D-Ausgabevorrichtung 1 gemäß Figur 1 mit einer an einem Tisch 25 sitzenden Person 26 in einer Ansicht von der Seite. Die Oberseite der Visualisierungseinrichtung 7 ist horizontal zur Oberfläche des Tisches 25 und zum Boden ausgerichtet. Dies ist durch drei gestrichelte Linien dargestellt. Die Person 26 blickt gerade nach vorn und sieht dabei das Äußere eines von ihr untersuchten Objektes und das Innere des von ihr untersuchten Objektes in der Form eines 3D Bildes. Das 3D Bild wird von einem von der Person 26 gesteuerten Bildgebungssystem aufgenommen und an die Visualisierungseinrichtung 7 übermittelt. Um zwischen dem realen Arbeitsraum und dem virtuellen Raum zu wechseln, genügt es, wenn die Person 26 an der Visualisierungseinrichtung 7 vorbeischaut oder diese aus ihrem Blickfeld bewegt. Hierzu muss die Person lediglich ihren Kopf leicht drehen. Die Person 26 bewegt die Visualisierungseinrichtung 7 beispielsweise von Hand oder mittels eines oder mehrerer Motoren, die über eine Eingabeeinrichtung gesteuert werden. Gut zu erkennen ist, dass die Visualisierungseinrichtung 7 nicht am Kopf der Person 26 befestigt ist, sondern ausschließlich an dem Gelenkarm 2. Die Person 26 kann sich somit jederzeit von der Visualisierungseinrichtung 7 entfernen, ohne dass sie diese berühren müsste. Es genügt beispielsweise eine Kopfbewegung nach hinten oder zur Seite, um sich von der Visualisierungseinrichtung 7 zu lösen. Figur 17 zeigt die 3D-Ausgabevorrichtung gemäß Figur 1 mit einer an einem Tisch 25 stehenden Person 26 in einer Ansicht von der Seite. Gut zu erkennen ist, dass sich die Person 26 nicht nur jederzeit von der Visualisierungseinrichtung 7 entfernen kann, sondern dass sie auch jederzeit eine andere Körperhaltung annehmen kann, indem sie von der in Figur 9 dargestellten sitzenden Position in die in Figur 17 dargestellte stehende Position wechselt. Ist die 3D-Anzeigevorrichtung 1 mit Sensoren ausgestattet, so können der Gelenkarm 2 und die Visualisierungseinrichtung 7 auch den Bewegungen der Person 26 folgen. Bewegt die Person beispielsweise ihren Kopf nach vorn oder tritt näher an das Objekt heran, so könnte sich der Gelenkarm 2 und die Visualisierungseinrichtung 7 automatisch um die gleiche Strecke nach hinten bewegen.

### Bezugszahlen:

- 1: 3D Ausgabevorrichtung
- 2: Gelenkarm 3
- 3:
- 3a: Erster Gelenkarmabschnitt
- 3b: Zweiter Gelenkarmabschnitt
- 3c: Dritter Gelenkarmabschnitt
- 4: Position des Basisgelenks
- 5: Gelenkarmbasis
- 6: Position des Kopfgelenks
- 7: Visualisierungseinrichtung
- 8: Monitorgehäuse
- 9: linker Monitor
- 10: rechter Monitor
- 11: linkes Sichtfenster
- 12: rechtes Sichtfenster
- 13: Sichtfenster-Gehäusewand
- 14: Nasenmulde
- 15: Streulicht-Schutzblende
- 16:
- 17: Kopplungsteil
- 18:
- 18a: erstes Kopfdrehgelenk
- 18b: zweites Kopfdrehgelenk
- 18c: drittes Kopfdrehgelenk
- 19: Schaft
- 20:
- 20a: Position des ersten Zwischendrehgelenks
- 20b: Position des zweites Zwischendrehgelenks
- 21: Säule
- 22: Geräteplatten
- 22: Verbindungsstück
- 23: Stift
- 24: Platte
- 25: Tisch
- 26: Person
- 27:
- 28: Standfuß
- 29: Doppellenkrollen
- a: Drehbewegung des Gelenks der Gelenkarmbasis
- b: Drehbewegung des Kopfgelenks
- c: Drehbewegung des ersten Zwischendrehgelenks
- d: Drehbewegung des zweiten Zwischendrehgelenks
- e: Drehbewegung des ersten Kopfdrehgelenks
- f: Drehbewegung des zweiten Kopfdrehgelenks
- g: Drehbewegung des dritten Kopfdrehgelenks

## Patentansprüche

1. 3D Ausgabevorrichtung (1) zur stereoskopischen Bildwiedergabe, mit einem Gelenkarm (2),
wobei der Gelenkarm (2) mit einem Basisgelenk ausgestattet ist, über welches der Gelenkarm (2) an einer Gelenkarmbasis (5) beweglich aufgenommen ist, und wobei der Gelenkarm (2) mit einem Kopfgelenk ausgestattet ist,
mit einer Visualisierungseinrichtung (7), welche über das Kopfgelenk beweglich an dem Gelenkarm (2) angeordnet ist,
mit einem Monitorgehäuse (8) der Visualisierungseinrichtung (7),
mit einem linken Monitor (9) und einem rechten Monitor (10), welche in dem Monitorgehäuse (8) angeordnet sind,
wobei das Monitorgehäuse (8) mit einem dem linken Monitor (9) zugeordneten linken Sichtfenster (11) ausgestattet ist, über welches der linke Monitor (9) von außen einsehbar ist,
wobei das Monitorgehäuse (8) mit einem dem rechten Monitor (10) zugeordneten rechten Sichtfenster (12) ausgestattet ist, über welches der rechte Monitor (10) von außen einsehbar ist,
wobei der Abstand zwischen dem linken Sichtfenster (11) und dem rechten Sichtfenster (12) einem typischen Augenabstand einer Person entspricht,
**dadurch gekennzeichnet, dass**
das Kopfgelenk ein erstes Kopfdrehgelenk (18a), ein zweites Kopfdrehgelenk (18b), ein drittes Kopfdrehgelenk (18c) und ein Kopplungsteil (17) umfasst, wobei das Kopplungsteil (17) das zweite Kopfdrehgelenk (18b) mit dem dritten Kopfdrehgelenk (18c) verbindet,
wobei die Drehachsen des ersten Kopfdrehgelenks (18a) und des zweiten Kopfdrehgelenks (18b) senkrecht zueinander stehen und wobei das dritte Kopfdrehgelenk (18c) eine Drehbewegung der Visualisierungsvorrichtung (7) um die Längsachse der länglich ausgebildeten Visualisierungsvorrichtung (7) ermöglicht.

2. 3D Ausgabevorrichtung nach Anspruch 1, **dadurch gekennzeichnet, dass** das Monitorgehäuse (8) eine Sichtfenster-Gehäusewand (13) aufweist, in welcher das linke und rechte Sichtfenster (11, 12) angeordnet sind, und dass die Sichtfenster-Gehäusewand (13) eine an die Kopfform einer Person angepasste Krümmung aufweist.

3. 3D Ausgabevorrichtung nach Anspruch 2, **dadurch gekennzeichnet, dass** die Sichtfenster-Gehäusewand (13) eine Nasenmulde (14) zwischen dem linken und rechten Sichtfenster (11, 12) aufweist.

4. 3D-Ausgabevorrichtung nach einem der vorhergehenden Ansprüche **dadurch gekennzeichnet, dass** das Monitorgehäuse (8) mit mindestens einer Streulicht-Schutzblende (15) ausgestattet ist, welche das Eindringen von Streulicht in das linke und rechte Sichtfenster (11, 12) reduziert.

5. 3D-Ausgabevorrichtung nach einem der vorhergehenden Ansprüche, **dadurch gekennzeichnet, dass** das Monitorgehäuse (8) einen linken Gehäuseabschnitt aufweist, welcher mit dem linken Monitor (9) und mit dem linken Sichtfenster (11) ausgestattet ist, dass das Monitorgehäuse (8) einen rechten Gehäuseabschnitt aufweist, welcher mit dem rechten Monitor (10) und dem rechten Sichtfenster (12) ausgestattet ist, und dass der linke Gehäuseabschnitt (16) von dem rechten Gehäuseabschnitt (17) zumindest abschnittsweise abgetrennt ist, derart dass über das linke Sichtfenster (11) nur der linke Monitor (9) einsehbar ist und über das rechte Sichtfenster (12) nur der rechte Monitor (10).

6. 3D Ausgabevorrichtung nach einen der vorherigen Ansprüche **dadurch gekennzeichnet, dass** das Kopfgelenk (6) ein Kopfdrehgelenk (18a, 18b, 18c) umfasst, über das die Visualisierungseinrichtung (7) um eine Drehachse beweglich an dem Gelenkarm (2) gelagert ist und sich ihrer Gewichtskraft folgend stets horizontal ausrichtet, wobei eine sich durch das Zentrum des linken Sichtfensters (11) und durch das Zentrum des rechten Sichtfensters (12) erstreckende Gerade horizontal verläuft.

7. 3D Ausgabevorrichtung nach Anspruch 6, **dadurch gekennzeichnet, dass** das Kopfdrehgelenk (18a, 18b, 18c) einen an dem Gelenkarm (2) angeordneten Stift (23) und eine an der Visualisierungseinrichtung (7) angeordnete Lagerschale aufweist, welche den Stift (23) umgreift.

8. 3D Ausgabevorrichtung nach einem der vorhergehenden Ansprüche, **dadurch gekennzeichnet, dass** der Gelenkarm (2) mindestens zwei Gelenkarmabschnitte (3a, 3b, 3c, 3d) aufweist, die über ein Zwischendrehgelenk (20a, 20b) miteinander beweglich verbunden sind.

9. 3D Ausgabevorrichtung nach einem der vorherigen Ansprüche **dadurch gekennzeichnet, dass** die Gelenkarmbasis (5) fahrbar ist.

10. 3D Ausgabevorrichtung nach einem der vorhergehenden Ansprüche **dadurch gekennzeichnet, dass** die Gelenkarmbasis (5) mit einer Befestigungseinrichtung ausgestattet ist, mit der der Gelenkarm (2) an einer Wand oder Decke eines Raumes oder an einem Objekt befestigbar ist.

11. 3D Ausgabevorrichtung nach einem der vorhergehenden Ansprüche, **dadurch gekennzeichnet, dass** sie mit mindestens einem Antrieb ausgestattet ist, welcher die Ausrichtung des Gelenkarms (2) und/ oder der Visualisierungseinrichtung (7) einstellt.

12. 3D Ausgabevorrichtung nach Anspruch 11, **dadurch gekennzeichnet, dass** der Antrieb mit einer Steuerungseinrichtung ausgestattet ist.

13. 3D Ausgabevorrichtung nach Anspruch 12, **dadurch gekennzeichnet, dass** die Steuerungseinrichtung eine Sprachsteuerung umfasst.

14. 3D Ausgabevorrichtung nach Anspruch 12 oder 13, **dadurch gekennzeichnet, dass** die Steuerungseinrichtung eine Bewegungssteuerung umfasst, welche Bewegungen eines Benutzers erfasst, diese auswertet und den Antrieb entsprechend steuert.

15. 3D Ausgabevorrichtung nach einem der Ansprüche 12 bis 14, **dadurch gekennzeichnet, dass** die Steuerungseinrichtung mit einer manuell betätigbaren Eingabeeinrichtung ausgestattet ist.

16. 3D Ausgabevorrichtung nach einem der vorhergehenden Ansprüche, **dadurch gekennzeichnet, dass** sie mit einer Feststelleinrichtung ausgestattet ist, mit der eine eingestellte Ausrichtung des Gelenkarms (2) und/ oder der Visualisierungseinrichtung (7) feststellbar ist.

17. 3D Ausgabevorrichtung nach einem der vorhergehenden Ansprüche, **dadurch gekennzeichnet, dass** der Gelenkarm (2) mit einem Griff ausgestattet, mit dem die Ausrichtung des Gelenkarms (2) manuell einstellbar ist.

18. 3D-Ausgabevorrichtung nach einem der vorhergehenden Ansprüche **dadurch gekennzeichnet, dass** der Gelenkarm (2) und/ oder die Visualisierungseinrichtung (7) mit einem austauschbaren Hygiene-Aufsatz ausgestattet ist.

19. 3D Ausgabevorrichtung nach einem der vorherigen Ansprüche **dadurch gekennzeichnet, dass** sie mit einem Bildgebungssystem ausgestattet ist.

## Claims

1. 3D output device (1) for stereoscopic image reproduction,
with an articulated arm (2),
wherein the articulated arm (2) is provided with a base joint, via which the articulated arm (2) is movably received on an articulated arm base (5), and wherein the articulated arm (2) is provided with a head joint,
with a visualization device (7), which is arranged movably on the articulated arm (2) by the head joint,
with a monitor housing (8) of the visualization device (7),
with a left monitor (9) and a right monitor (10) which are arranged in the monitor housing (8),
wherein the monitor housing (8) is provided with a left viewing window (11), which is assigned to the left monitor (9) and through which the left monitor (9) can be viewed from the outside,
wherein the monitor housing (8) is provided with a right viewing window (12), assigned with the right monitor (10), through which the right monitor (10) can be viewed from the outside,
wherein the distance between the left viewing window (11) and the right viewing window (12) corresponds to a typical interocular distance of a person,
**characterized in that**
the head joint comprises a first head pivot joint (18a), a second head pivot joint (18b), a third head pivot joint (18c) and a coupling member (17), whereby the coupling member (17) connects the second head pivot joint (18b) to the third head pivot joint (18c),
whereby the geometric axes of rotation of the first head pivot joint (18a) and the second head pivot joint (18b) are perpendicular to each other,
and whereby the third head pivot joint (18c) allows the visualization device (7) to be rotated about the longitudinal axis L of the present elongated visualization device (7).

2. 3D output device according to claim 1, **characterized in that** the monitor housing (8) has a viewing window housing wall (13) in which the left and right viewing windows (11, 12) are arranged, and that the viewing window housing wall (13) has a curvature adapted to the shape of a person's head.

3. 3D output device according to claim 2, **characterized in that** the viewing window housing wall (13) has a nose trough (14) between the left and right viewing windows (11, 12).

4. 3D output device according to one of the preceding claims, **characterized in that** the monitor housing (8) is provided with at least one stray light protection shield (15) which reduces the penetration of stray light into the left and right viewing windows (11, 12).

5. 3D output device according to one of the preceding claims, **characterized in that** the monitor housing (8) has a left housing section provided with the left monitor (9) and with the left viewing window (11), and that the monitor housing (8) has a right housing section provided with the right monitor (10) and with the right viewing window (12), and **in that** the left housing section (16) is separated at least in sections from the right housing section (17) in such a way that only the left monitor (9) can be viewed through the left viewing window (11) and only the right monitor (10) can be viewed through the right viewing window (12).

6. 3D output device according to one of the preceding claims, **characterized in that** the head joint (6) comprises a head pivot joint (18a, 18b, 18c) by means of which the visualization device (7) is mounted on the articulated arm (2) so as to be movable about an axis of rotation and aligns itself horizontally according to its weight, wherein a straight line extending through the center of the left viewing window (11) and through the center of the right viewing window (12) is horizontal.

7. 3D output device according to claim 6, **characterized in that** the head pivot joint (18a, 18b, 18c) comprises a pin (23) arranged on the articulated arm (2) and a bearing shell arranged on the visualization device (7) surrounding the pin (23).

8. 3D output device according to one of the preceding claims, **characterized in that** the articulated arm (2) comprises at least two articulated arm sections (3a, 3b, 3c, 3d) which are movably connected to each other by an intermediate pivot joint (20a, 20b).

9. 3D output device according to one of the previous claims, **characterized in that** the articulated arm base (5) is movable.

10. 3D output device according to one of the preceding claims, **characterized in that** the articulated arm base (5) is provided with a fastening device with which the articulated arm (2) can be fastened to a wall or ceiling of a room or to an object.

11. 3D output device according to one of the preceding claims, **characterized in that** it is provided with at least one drive which adjusts the orientation of the articulated arm (2) and/or of the visualization device (7).

12. 3D output device according to claim 11, **characterized in that** the drive is provided with a control device.

13. 3D output device according to claim 12, **characterized in that** the control device comprises a voice control.

14. 3D output device according to claim 12 or 13, **characterized in that** the control device comprises a motion control which detects movements of a user, evaluates them and controls the drive accordingly.

15. 3D output device according to one of claims 12 to 14, **characterized in that** the control device is equipped with a manually operable input device.

16. 3D output device according to one of the preceding claims, **characterized in that** it comprises a locking device with which a set alignment of the articulated arm (2) and/or of the visualization device (7) can be locked.

17. 3D output device according to one of the preceding claims, **characterized in that** the articulated arm (2) is provided with a handle for manually adjusting the orientation of the articulated arm (2).

18. 3D output device according to one of the preceding claims, **characterized in that** the articulated arm (2) and/or the visualization device (7) is provided with a replaceable hygiene attachment.

19. 3D output device according to one of the preceding claims, **characterized in that** it is provided with an imaging system.

## Revendications

1. Dispositif de sortie 3D (1) pour la reproduction d'images stéréoscopiques, comportant un bras articulé (2), dans lequel le bras articulé (2) est équipé d'une articulation de base par l'intermédiaire de laquelle le bras articulé (2) est reçu de manière à être mobile sur une base pour bras articulé (5), et dans lequel le bras articulé (2) est équipé d'une articulation de tête, comportant un dispositif de visualisation (7) qui est disposé de manière mobile sur le bras articulé (2) par l'intermédiaire de l'articulation de tête, comportant un boîtier de moniteurs (8) du dispositif de visualisation (7), comportant un moniteur gauche (9) et un moniteur droit (10) qui sont disposés dans le boîtier de moniteurs (8), dans lequel le boîtier de moniteurs (8) est équipé d'une fenêtre d'observation gauche (11) associée au moniteur gauche (9), par l'intermédiaire de laquelle le moniteur gauche (9) peut être observé de l'extérieur, dans lequel le boîtier de moniteurs (8) est équipé d'une fenêtre d'observation droite (12) associée au moniteur droit (10), par l'intermédiaire de laquelle le moniteur droit (10) peut être observé de l'extérieur, dans lequel la distance entre la fenêtre d'observation gauche (11) et la fenêtre d'observation droite (12) correspond à une distance typique entre les yeux d'une personne, **caractérisé en ce que**
l'articulation de tête comprend une première articulation rotative de tête (18a), une deuxième articulation rotative de tête (18b), une troisième articulation rotative de tête (18c) et une partie d'accouplement (17), dans lequel la partie d'accouplement (17) relie la deuxième articulation rotative de tête (18b) à la troisième articulation rotative de tête (18c), dans lequel les axes de rotation de la première articulation rotative de tête (18a) et de la deuxième articulation rotative de tête (18b) sont perpendiculaires l'un à l'autre et dans lequel la troisième articulation rotative de tête (18c) permet un mouvement de rotation du dispositif de visualisation (7) autour de l'axe longitudinal du dispositif de visualisation (7) réalisé sous forme allongée.

2. Dispositif de sortie 3D selon la revendication 1, **caractérisé en ce que** le boîtier de moniteurs (8) présente une paroi de boîtier à fenêtres de visualisation (13) dans laquelle sont disposées les fenêtres de visualisation gauche et droite (11, 12), et **en ce que** la paroi de boîtier à fenêtres de visualisation (13) présente une courbure adaptée à la forme de la tête d'une personne.

3. Dispositif de sortie 3D selon la revendication 2, **caractérisé en ce que** la paroi de boîtier à fenêtres d'observation (13) présente un creux pour nez (14) entre les fenêtres d'observation gauche et droite (11, 12).

4. Dispositif de sortie 3D selon l'une des revendications précédentes, **caractérisé en ce que** le boîtier de moniteurs (8) est équipé d'au moins un écran de protection contre la lumière parasite (15) qui réduit la pénétration de la lumière parasite dans les fenêtres de visualisation gauche et droite (11, 12).

5. Dispositif de sortie 3D selon l'une des revendications précédentes, **caractérisé en ce que** le boîtier de moniteurs (8) présente une section de boîtier gauche qui est équipée du moniteur gauche (9) et de la fenêtre de visualisation gauche (11), **en ce que** le boîtier de moniteurs (8) présente une section de boîtier droite qui est équipée du moniteur droit (10) et de la fenêtre d'observation droite (12), et **en ce que** la section de boîtier gauche (16) est séparée au moins dans certaines sections de la section de boîtier droite (17), de telle sorte que seul le moniteur gauche (9) est visible par l'intermédiaire de la fenêtre d'observation gauche (11) et que seul le moniteur droit (10) est visible par l'intermédiaire de la fenêtre d'observation droite (12).

6. Dispositif de sortie 3D selon l'une des revendications précédentes, **caractérisé en ce que** l'articulation de tête (6) comprend une articulation rotative de tête (18a, 18b, 18c) par l'intermédiaire de laquelle le dispositif de visualisation (7) est monté sur le bras articulé (2) de manière à être mobile autour d'un axe de rotation et s'oriente toujours horizontalement en suivant son poids, dans lequel une droite passant par le centre de la fenêtre de visualisation gauche (11) et par le centre de la fenêtre de visualisation droite (12) s'étend en étant horizontale.

7. Dispositif de sortie 3D selon la revendication 6, **caractérisé en ce que** l'articulation rotative de tête (18a, 18b, 18c) présente une broche (23) disposée sur le bras articulé (2) et une coque de support disposée sur le dispositif de visualisation (7) et entourant la broche (23).

8. Dispositif de sortie 3D selon l'une des revendications précédentes, **caractérisé en ce que** le bras articulé (2) présente au moins deux sections de bras articulé (3a, 3b, 3c, 3d) qui sont reliées entre elles de manière à être mobiles par l'intermédiaire d'une articulation rotative intermédiaire (20a, 20b).

9. Dispositif de sortie 3D selon l'une des revendications précédentes, **caractérisé en ce que** la base pour bras articulé (5) est mobile.

10. Dispositif de sortie 3D selon l'une des revendications précédentes, **caractérisé en ce que** la base pour bras articulé (5) est équipée d'un dispositif de fixation au moyen duquel le bras articulé (2) peut être fixé sur un mur ou un plafond d'une pièce ou sur un objet.

11. Dispositif de sortie 3D selon l'une des revendications précédentes, **caractérisé en ce qu'**il est équipé d'au moins un entraînement qui règle l'orientation du bras articulé (2) et/ou du dispositif de visualisation (7).

12. Dispositif de sortie 3D selon la revendication 11, **caractérisé en ce que** l'entraînement est équipé d'un dispositif de commande.

13. Dispositif de sortie 3D selon la revendication 12, **caractérisé en ce que** le dispositif de commande comprend une commande vocale.

14. Dispositif de sortie 3D selon la revendication 12 ou 13, **caractérisé en ce que** le dispositif de commande comprend une commande de mouvement qui détecte les mouvements d'un utilisateur, les évalue et commande l'entraînement en conséquence.

15. Dispositif de sortie 3D selon l'une des revendications 12 à 14, **caractérisé en ce que** le dispositif de commande est équipé d'un dispositif d'entrée actionnable manuellement.

16. Dispositif de sortie 3D selon l'une des revendications précédentes, **caractérisé en ce qu'**il est équipé d'un dispositif de blocage au moyen duquel une orientation réglée du bras articulé (2) et/ou du dispositif de visualisation (7) peut être bloquée.

17. Dispositif de sortie 3D selon l'une des revendications précédentes, **caractérisé en ce que** le bras articulé (2) est équipé d'une poignée au moyen de laquelle l'orientation du bras articulé (2) peut être réglée manuellement.

18. Dispositif de sortie 3D selon l'une des revendications précédentes, **caractérisé en ce que** le bras articulé (2) et/ou le dispositif de visualisation (7) sont équipés d'un embout hygiénique interchangeable.

19. Dispositif de sortie 3D selon l'une des revendications précédentes, **caractérisé en ce qu'**il est équipé d'un système d'imagerie.
